# EUROPEAN PATENT APPLICATION

(11) **EP 2 624 205 A1**
(43) Date of publication of application: **07.08.2013**
(21) Application number: 10857844.4
(22) Date of filing: 30.09.2010
(51) Int. Cl.: G06T 7/00, A61B 5/117

(54) **REGISTRATION PROGRAM, REGISTRATION DEVICE, AND REGISTRATION METHOD**

(71) Applicant: Fujitsu Frontech Limited, Inagi-shi Tokyo 206-8555 (JP)
(72) Inventor: KAMATA, Hideo, Inagi-shi Tokyo 206-8555 (JP); MINAGAWA, Akitaka, Inagi-shi Tokyo 206-8555 (JP); HIGASHIURA, Yasuyuki, Inagi-shi Tokyo 206-8555 (JP); KASUGAI, Kentarou, Inagi-shi Tokyo 206-8555 (JP); IDE, Katsumi, Inagi-shi Tokyo 206-8555 (JP)
(74) Representative: HOFFMANN EITLE
(86) International application number: PCT/JP2010/067044
(87) International publication number: WO 2012/042631

(57) **Abstract**

To provide a registration program, a registration apparatus, and a registration method which are capable of performing appropriate template registration by rejecting inappropriate template registration.

A registration apparatus (10) acquires image information of a living body photographed by a sensor unit and distance information acquired when photographing the living body from the sensor unit, and extracts image information which is to serve as a template. The registration apparatus (10) acquires a plurality of items of the image information by an image information acquisition means (10a), and acquires distance information associated with each item of the image information item by a distance information acquisition means (10b). A template candidate extraction means (10c) groups the plurality of image information items according to the distance information associated with each image information item. A template extraction means (10d) mutually evaluates the image information items extracted by the template candidate extraction means (10c) to extract image information to be registered as a template for each group.

## Description

### Technical Field

The present invention relates to a registration program, a registration apparatus, and a registration method, for registering biometric information used for verification in advance so as to authenticate an individual by making use of biometric information.

### Background Art

A human body includes biometric information items which make it possible to identify an individual, and some of the biometric information items are used as information for identifying and authenticating the individual. For example, it is known that the biometric information items which are considered to be capable of being used for authentication include fingerprints, eye retinas and irises, a face, vessels, DNA (Deoxyribo Nucleic Acid), and so forth.

In recent years, with the development of a biometric authentication technique, there have been provided various types of apparatuses for authenticating an individual by recognizing biometric features of part of a human body. In this biometric authentication, the authentication of an individual is performed by comparing biometric information (registered template) acquired during registration thereof and biometric information acquired during authentication.

To improve the accuracy of individual authentication using such biometric information, it is desirable to acquire accurate biometric information, and therefore the authentication apparatuses acquire biometric information under sensing conditions made identical. However, for example, if a registered template is an inappropriate one, such as a template registered by an incorrect posture, even when the registered template and the biometric information acquired during authentication are compared, it is not possible to obtain a proper result of verification.

To eliminate this problem, there has been proposed an authentication system in which a template is registered based on not biometric information acquired by only one operation, but a plurality of biometric information items acquired by more than one operation (see e.g. PTL 1). According to this authentication system, a processing unit acquires biometric information on an identical living body from a detection unit more than once, and determines a degree of mutual similarity between respective biometric feature data items obtained from the plurality of acquired biometric information items. Then, a plurality of biometric feature data items which are high in similarity are registered in a storage unit.

### Citation List

### Patent literature

PTL 1: Japanese Laid-Open Patent Publication No. 2006-6753

### Summary of Invention

### Technical Problem

However, there are cases where it is not possible to obtain a proper result of verification even by the template registration based on the plurality of biometric information items, and these inconveniences are mostly caused by insufficient recognition of an appropriate posture or conditions during registration.

Such inappropriately registered template requires re-registration, and if the service is provided by a financial institution, such as a bank, it is required to perform an operation for re-registering the template at a teller's window. The user has to go to a place for registration operation (banking office in most cases), and the financial institution suffers lowering of customer satisfaction and degradation of customer service.

In many companies, including financial institutions, non-regular employees are sometimes assigned as tellers, and some of them are not familiarized with the operation of template registration, and hence inappropriate template registration is sometimes overlooked.

Further, even for personal use, repetition of the operation of template registration results in lowered user's customer satisfaction and degraded service, which is one of dissatisfactions with the biometric authentication.

The present invention has been made in view of these points, and an object thereof is to provide a registration program, a registration apparatus, and a registration method, which are capable of performing appropriate template registration by rejecting inappropriate template registration.

### Solution to Problem

To solve the above problem, a registration program for causing a computer to execute processing for registering a template for use in biometric authentication causes the computer to execute the following processing of:
acquiring, from a sensor unit which is capable of outputting image information of a photographed living body and distance information acquired when photographing the living body, a plurality of items of the image information, acquiring the distance information associated with each item of the image information from the sensor unit, grouping the plurality of items of the image information according to the distance information associated with each item of the image information, and mutually evaluating the image information on a group-by-group basis according to the distance information to thereby extract an item of the image information, which is to serve as a template candidate, and mutually evaluating extracted items of the image information to thereby extract an item of the image information, which is to serve as a template.

Further, to solve the above problems, a registration apparatus that registers a template for use in biometric authentication includes image information acquisition means, distance information acquisition means, template candidate extraction means, and template extraction means.

The image information acquisition means acquires, from a sensor unit which is capable of outputting image information of a photographed living body and distance information acquired when photographing the living body, a plurality of items of the image information. The distance information acquisition means acquires the distance information associated with each item of the image information from the sensor unit. The template candidate extraction means groups the plurality of items of the image information according to the distance information associated with each item of the image information, and mutually evaluates the image information on a group-by-group basis according to the distance information to thereby extract an item of the image information, which is to serve as a template candidate. The template extraction means for mutually evaluating extracted items of the image information to thereby extract an item of the image information, which is to serve as a template.

Further, to solve the above problems, a method of registering a template for use in biometric authentication includes acquiring, from a sensor unit which is capable of outputting image information of a photographed living body and distance information acquired when photographing the living body, a plurality of items of the image information, acquiring the distance information associated with each item of the image information from the sensor unit, grouping the plurality of items of the image information according to the distance information associated with each item of the image information, and mutually evaluating the image information on a group-by-group basis according to the distance information to thereby extract an item of the image information, which is to serve as a template candidate, and mutually evaluating extracted items of the image information to thereby extract an item of the image information, which is to serve as a template.

### Advantageous Effects of Invention

According to the above-described registration program, registration apparatus, and registration method, it is possible to perform appropriate template registration by rejecting inappropriate template registration.

The above and other objects, features and advantages of the present invention will become apparent from the following description when taken in conjunction with the accompanying drawings which illustrate preferred embodiments of the present invention by way of example.

### Brief Description of Drawings

[FIG. 1] FIG. 1 illustrates the configuration of a registration apparatus according to a first embodiment.
[FIG. 2] FIG. 2 illustrates the configuration of an authentication system according to a second embodiment.
[FIG. 3] FIG. 3 illustrates template registration according to the second embodiment.
[FIG. 4] FIG. 4 illustrates the configuration of a registration apparatus according to the second embodiment.
[FIG. 5] FIG. 5 illustrates the configuration of a sensor unit according to the second embodiment.
[FIG. 6] FIG. 6 illustrates an example of the hardware configuration of the registration apparatus according to the second embodiment.
[FIG. 7] FIG. 7 is a flowchart of a template registration process according to the second embodiment.
[FIG. 8] FIG. 8 is a flowchart of a photographing process according to the second embodiment.
[FIG. 9] FIG. 9 is a table indicative of similarity scores and similarity grouping of far-distance images, according to the second embodiment.
[FIG. 10] FIG. 10 is a table indicative of similarity scores and similarity grouping of medium-distance images, according to the second embodiment.
[FIG. 11] FIG. 11 is a table indicative of similarity scores and similarity grouping of near-distance images, according to the second embodiment.
[FIG. 12] FIG. 12 illustrates the configuration of a verification apparatus according to the second embodiment.
[FIG. 13] FIG. 13 is a flowchart of a verification process according to the second embodiment.
[FIG. 14] FIG. 14 illustrates registered templates and verification images according to the second embodiment.
[FIG. 15] FIG. 15 illustrates template registration according to a third embodiment.
[FIG. 16] FIG. 16 illustrates template registration according to a fourth embodiment.
[FIG. 17] FIG. 17 illustrates registered templates and a verification image according to a fifth embodiment.

### Description of Embodiments

Embodiments of the present invention will be explained below with reference to the accompanying drawings.

### [First Embodiment]

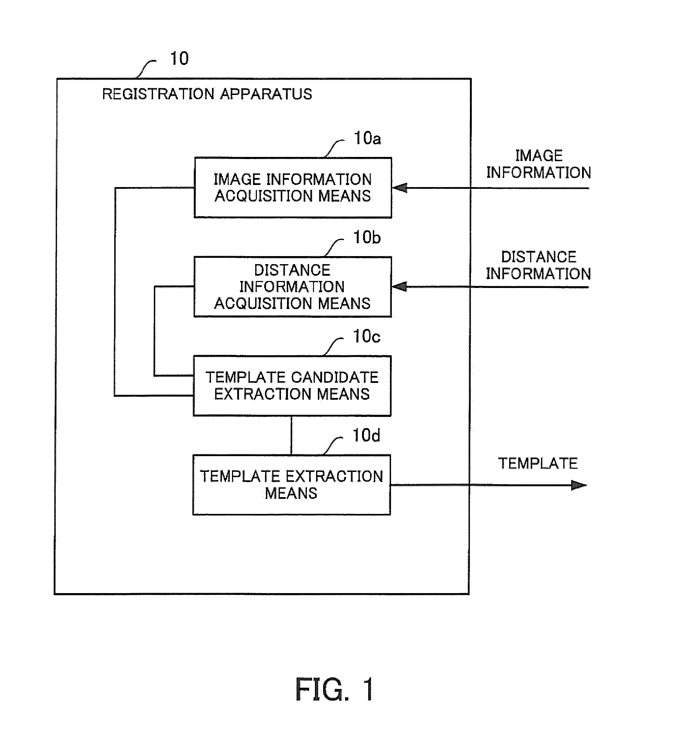

First, a description will be given of a registration apparatus according to a first embodiment with reference to FIG. 1. FIG. 1 illustrates the configuration of the registration apparatus according to the first embodiment.

The registration apparatus 10 acquires image information of a living body photographed by a sensor unit and distance information acquired when photographing the living body from the sensor unit, and extracts image information which is to serve as a template. The sensor unit, including an image sensor and a distance measurement sensor, photographs a living body as an object, generates image information of the living body and distance information at the time of photographing the living body, and outputs the generated image information and distance information to the registration apparatus 10. The image information is image data of the living body, and is generated according to a predetermined image format. The distance information is information indicative of a distance between the sensor unit and the living body at the time of photographing. The template is data acquired from the living body in advance for use in verification of the living body.

The registration apparatus 10 includes an image information acquisition means 10a, a distance information acquisition means 10b, a template candidate extraction means 10c, and a template extraction means 10d. The image information acquisition means 10a acquires a plurality of image information items. The distance information acquisition means 10b acquires distance information (photographing distance) associated with each image information item acquired by the image information acquisition means 10a.

The template candidate extraction means 10c groups the plurality of image information items according to the distance information associated with each image information item. By this grouping, the plurality of image information items are grouped into e.g. three groups of "far", "medium,", and "near" according to the photographing distance from the sensor unit. The template candidate extraction means 10c mutually evaluates the image information items for each group, grouped according to the distance information, and thereby extracts image information which is to serve as a template candidate. The mutual evaluation is performed such that one of the image information items is set as a reference image, the rest of the image information items are set as objects to be evaluated, and evaluation values are determined according to a predetermined evaluation method with respect all of combination of image information items, while changing the reference image. As a result of the mutual evaluation, for example, two image information items are extracted from the group set as "far", two image information items are extracted from the group set as "medium", and two image information items are extracted from the group set as "near", whereby total six image information items are extracted as template candidates.

The template extraction means 10d mutually evaluates the image information items extracted by the template candidate extraction means 10c. Based on the result of this evaluation, the template extraction means 10d extracts image information which is to serve as a template. For example, the template extraction means 10d mutually evaluates the six images extracted from the three groups of "far", "medium", and "near" without discriminating between the groups. Based on the result of this evaluation, the template extraction means 10d extracts an image information item to be registered as a template for each of the groups of "far", "medium", and "near".

Thus, the registration apparatus 10 extracts image information items appropriate for template candidates for each distance, and thereafter extracts more appropriate image information items as a whole. As a result, if appropriate image information exists in the image information items for each distance, i.e. if any image information item indicates an appropriate posture during the process of registration operation, the image information appropriate for registration (which can be evaluated as most appropriate or nearly most appropriate for registration) is registered as a template.

Next, the registration apparatus will be more specifically described using a second embodiment.

### [Second Embodiment]

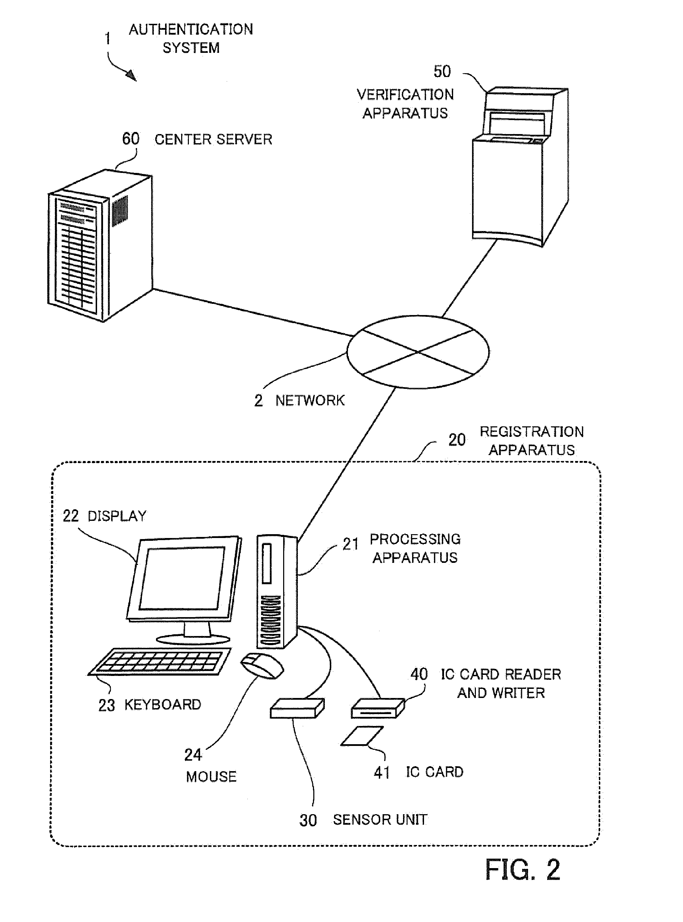

FIG. 2 illustrates an authentication system according to the second embodiment. Although in the second embodiment, the authentication system 1 is illustrated as an authentication system that performs authentication of an individual, using veins of a palm, by way of example, this is not limitative, but it is applicable to a system which performs the authentication using another portion of a living body for biometric feature detection.

The authentication system 1 is one which identifies and authenticates an individual by recognizing a biometric feature, and can be used for logging on to an information system or controlling the entrance and exit to and from a room. The authentication system 1 includes a registration apparatus 20, and a center server 60 and a verification apparatus 50 connected to the registration apparatus 20 via a network 2.

The center server 60 stores identification information for identifying individuals and biometric information (templates) registered in advance for biometric authentication in a manner associated with each other. The identification information for identifying an individual is unique ID (IDentification) information given to a user directly (e.g. a user number) or indirectly (e.g. a bank account number). The biometric information registered in advance includes feature information acquired by extracting a feature portion from image information, and encoded information acquired by encoding the image information or the feature information.

The verification apparatus 50 is one which performs biometric authentication when authenticating a user. The verification apparatus 50 is e.g. an ATM (Automated Teller Machine) installed in a financial institute, a management apparatus of a security area, or a personal computer which performs user authentication.

The registration apparatus 20 comprises a processing apparatus 21, a display 22, and a sensor unit 30. The registration apparatus 20 further comprises a keyboard 23, a mouse 24, an IC (Integrated Circuit) card reader and writer 40, and so forth, as required. The sensor unit 30, which includes an image pickup device, photographs an image of a palm of the user, and outputs the photographed image to the processing apparatus 21. The IC card reader and writer 40 reads and writes information of an IC card 41 of the user. The keyboard 23 and the mouse 24 receive user input operations.

Now, a description will be given of template registration for palm vein authentication, performed by the registration apparatus 20. A user requesting template registration inputs identification information (e.g. user ID) for identifying the user using the keyboard 23 and the mouse 24 or the IC card reader and writer 40. The registration apparatus 20 guides the user to the template registration via display using the display 22, and requests the user to input biometric information for template registration. The user holds a hand over the sensor unit 30 to thereby input the biometric information. The registration apparatus 20 having an image of the palm veins input therein as the biometric information generates a registered template from the input information, and stores the registered template in a storage section of the processing apparatus 21, a storage section of the center server 60, or a storage section of the IC card 41 of the user.

For example, if the registration apparatus 20 is one for personal use, which also functions as the verification apparatus, the registration apparatus 20 stores the registered template in the storage section of the processing apparatus 21 or the storage section of the IC card 41 of the user. Further, if the registration apparatus 20 is provided separately from the verification apparatus, the registration apparatus 20 stores the registered template in the storage section of the center server 60 or the storage section of the IC card 41. In this case, when performing biometric authentication, the registration apparatus 20 obtains the template from the storage section of the center server 60 or the storage section of the IC card 41 to thereby verify the input biometric information.

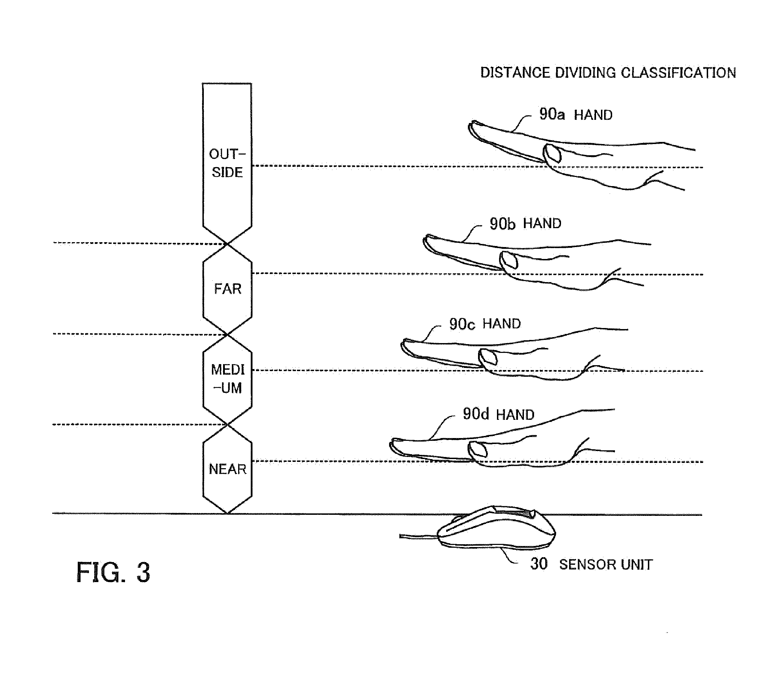

Next, a description will be given of a positional relationship between the sensor unit 30 and a hand at a time when performing template registration for palm vein authentication. FIG. 3 illustrates template registration according to the second embodiment.

The template registration for palm vein authentication is performed based on an image (image information) obtained by photographing an operation of holding a palm over the sensor unit 30. In general, the palm-holding over operation 30 is performed such that the hand comes closer to the sensor unit 30 from above. Further, the registration apparatus 20 guides a person to be registered on how to perform the palm-holding over operation 30 using an image displayed on the display 22 or a sound output from a speaker, not illustrated. Further, e.g. at a teller window of a financial institute, a teller guides the person to be registered on how to perform the palm-holding over operation 30.

The palm of the person to be registered is moved closer to the sensor unit 30 while being horizontally positioned to the sensor unit 30. To this end, the sensor unit 30, including a wide-angle lens, is capable of capturing a wide range of the palm as an object, in a photographable range.

Further, the sensor unit 30 include a distance measurement sensor and photographs the palm at distances within a predetermined range. For example, the hand of the person to be registered comes closer to the sensor unit 30, as indicated by hands 90a, 90b, 90c, and 90d. Assuming, for example, that a range of distance between the palm and the sensor unit 30 of 20 mm to 80 mm is the photographable range, the palm at a distance farther than 80 mm from the sensor unit 30 and the palm at a distance nearer than 20 mm from the sensor unit 30 are not photographed. Therefore, the palm of the hand 90a is not photographed, and the palms of the hands 90b, 90c, and 90d are photographed.

Each photographed image is associated with the distance information output from the distance measurement sensor, which makes it possible to group the images according to the distance such that the image of the hand 90b is an image of the "far" location, the image of the hand 90c is an image of the "medium" location, and the image of the hand 90d is an image of the "near" location. Threshold values used for grouping the images have reference values set in advance such that the values from 20 mm to 40 mm are set to "near", the values from 40 mm to 60 mm are set to "medium", and the values from 60 mm to 80 mm are set to "far". These reference values may be held by the sensor unit 30, and the distance information may be set as distance ranks (e.g. "far", "medium,", and "near"). When the sensor unit 30 outputs the distance information as a specific distance, these reference values are stored in the registration apparatus 20.

The sensor unit 30 acquires approximately fifteen images by photographing fifteen frames per second during a single operation of holding a palm over the sensor unit 30. Not all of the images thus acquired by the sensor unit 30 are suitable for the registered template because the person to be registered sometimes does not fully open his/her palm, tilts his/her palm, or rolls over his/her wrist during the process of the operation of holding the palm over the sensor unit 30.

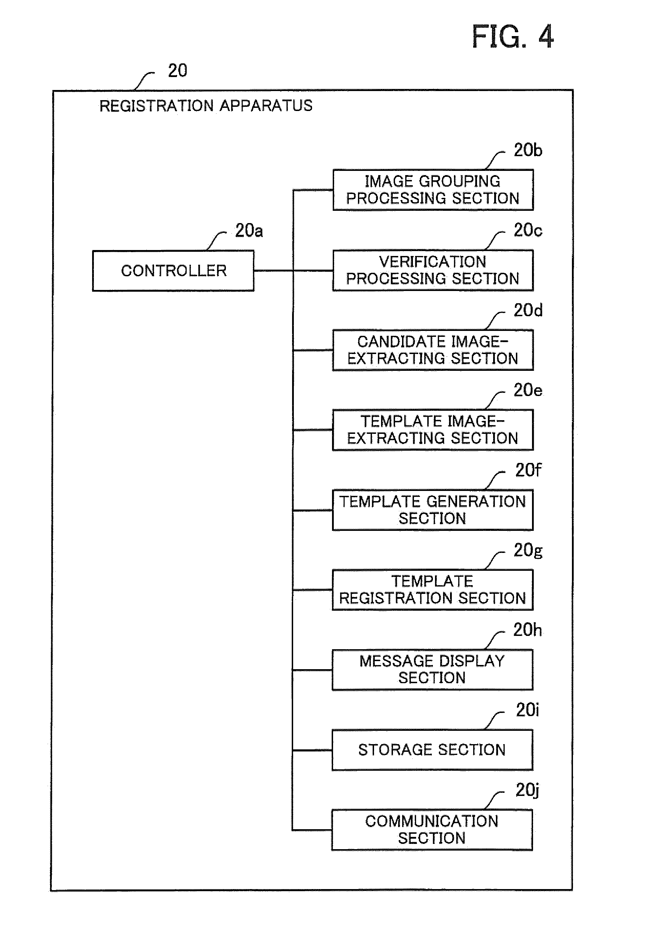

Next, a description will be given of the registration apparatus 20 which realizes a process for registering a template for palm vein authentication with reference to FIG. 4. FIG. 4 illustrates the registration apparatus according to the second embodiment.

The registration apparatus 20 includes a controller 20a, an image grouping processing section 20b, a verification processing section 20c, a candidate image-extracting section 20d, a template image-extracting section 20e, a template generation section 20f, a template registration section 20g, a message display section 20h, a storage section 20i, and a communication section 20j.

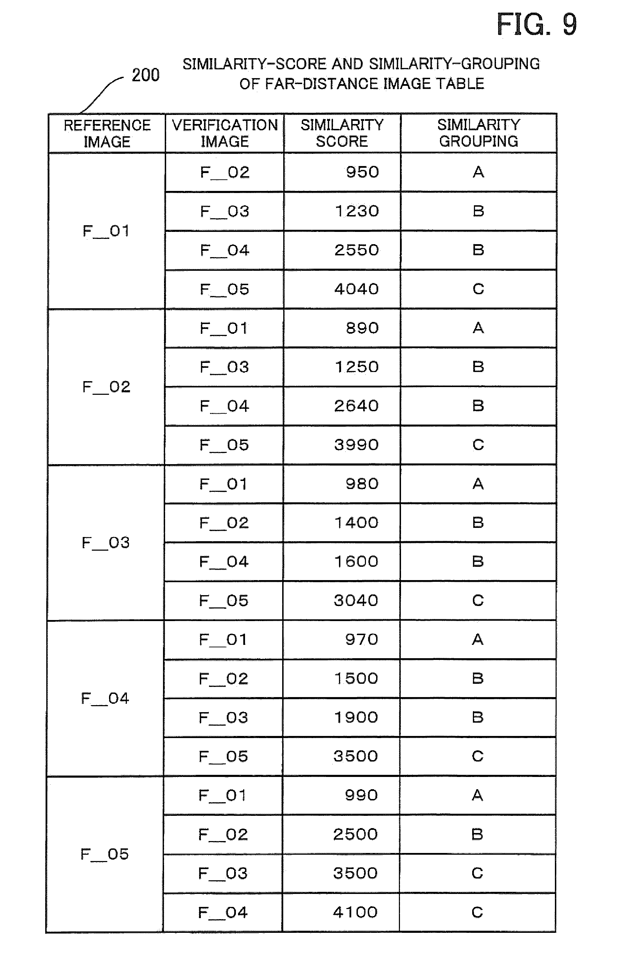

The controller 20a performs centralized control of the processing sections. The image grouping processing section 20b groups a plurality of image information items acquired from the sensor unit 30 on a predetermined distance range basis based on the distance information associated with each image information item also acquired from the sensor unit 30. The verification processing section 20c mutually verifies the plurality of image information items, and calculates a degree of similarity to perform mutual evaluation. The mutual evaluation will be described in detail hereinafter with reference to FIGS. 9 to 11.

The candidate image-extracting section 20d performs mutual evaluation by the verification processing section 20c on each image information item grouped by the image grouping processing section 20b in a manner discriminating between the groups to thereby extract image information as template candidates in each group. The template image-extracting section 20e performs mutual evaluation by the verification processing section 20c on the image information extracted from each group by the candidate image-extracting section 20d in a manner not discriminating between the groups to thereby extract image information which is to serve as a template in each group. The registration apparatus 20 thus extracts image information appropriate as template candidates for each distance, and then extracts more appropriate image information as a whole.

The template generation section 20f processes the image information extracted by the template image-extracting section 20e into a registered template. The template registration section 20g stores (registers) the registered template in the storage section of the processing apparatus 21, the storage section of the center server 60, or the storage section of the IC card 41 of the user.

The message display section 20h generates necessary messages for the person to be registered, such as a guidance on how to perform the palm-holding over operation 30, and a notification of whether or not the template registration is successful, and displays the generated messages on the display 22.

The storage section 20i stores and holds not only the image information and distance information acquired from the sensor unit 30 but also operation information, such as grouping data and similarity scores, and the generated templates. The communication section 20j performs communication with the sensor unit 30, communication with the IC card reader and writer 40, and communication with the center server 60 via the network 2.

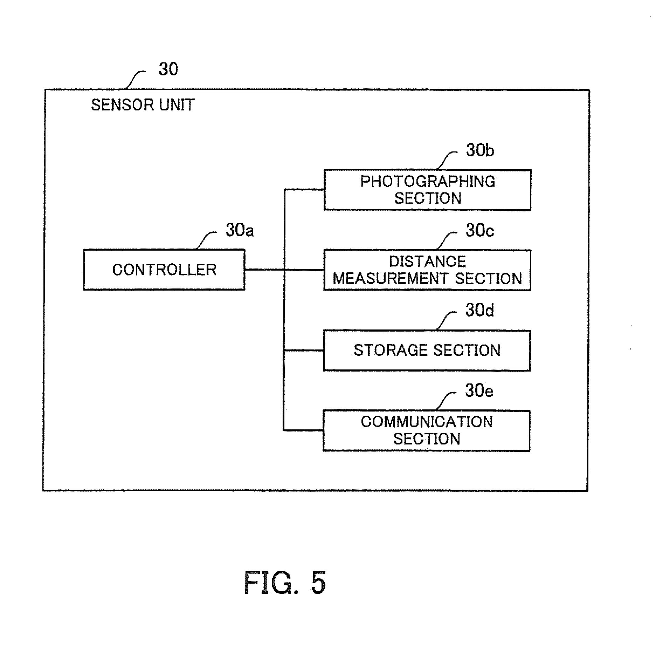

Next, a description will be given of the sensor unit 30 which realizes a process for acquiring the image information and distance information, and outputting the acquired image information and distance information to the registration apparatus 20 with reference to FIG. 5. FIG. 5 illustrates the sensor unit according to the second embodiment.

The sensor unit 30 includes a controller 30a, a photographing section 30b, a distance measurement section 30c, a storage section 30d, and a communication section 30e.

The controller 30a performs centralized control of the processing sections. The photographing section 30b acquires image information from a living body as an object. The photographing section 30b includes an image sensor which photographs a living body (e.g. CMOS (Complementary Metal Oxide Semiconductor) sensor or CCD (Charge Coupled Device) sensor), a condenser lens, and a plurality of near infrared ray-emitting devices (LEDs: Light Emitting Diodes) which irradiate an object. The near infrared ray-emitting devices are provided e.g. around the image sensor, and emit near infrared rays toward the object (upward), and the image sensor photographs the object irradiated with near infrared rays. The photographing section 30b is capable of continuously photographing an object, and photographs e.g. fifteen frames per second. The photographing speed may be changed by configuration. Further, the photographing timing may not be determined according to time, but according to distance from the object based on the output from the distance measurement section 30c. Note that the photographing section 30b is configured to be suitable for photographing palm veins, and when a living body portion other than the palm, such as an iris, is to be photographed, a configuration suitable for photographing the object may be employed.

The distance measurement section 30c acquires information on a distance from a living body as an object. The storage section 30d stores the image information acquired by the photographing section 30b and the distance information acquired by the distance measurement section 30c in a manner associated with each other. The communication section 30e is connected with the communication section 20j of the registration apparatus 20 to receive instructions from the registration apparatus 20 and transmit the image information and the distance information to the registration apparatus 20.

The image photographed by the sensor unit 30 is an image acquired by irradiating near infrared rays onto a living body (palm) as an object and photographing reflected light from the palm. Since hemoglobin in red cells flowing through veins of the palm has lost oxygen, the hemoglobin (reduced hemoglobin) has the property of absorbing near infrared rays in the vicinity of 700 nm to 1000 nm. Therefore, when near infrared rays are irradiated onto the palm, there is little reflection of the near infrared rays only from a portion where the veins extend, whereby it is possible to recognize positions of the veins by the degree of the intensity of the reflected light of the near infrared rays. Although the photographed image is made easy to extract characteristic information by using a specific light source, it becomes an achromatic image.

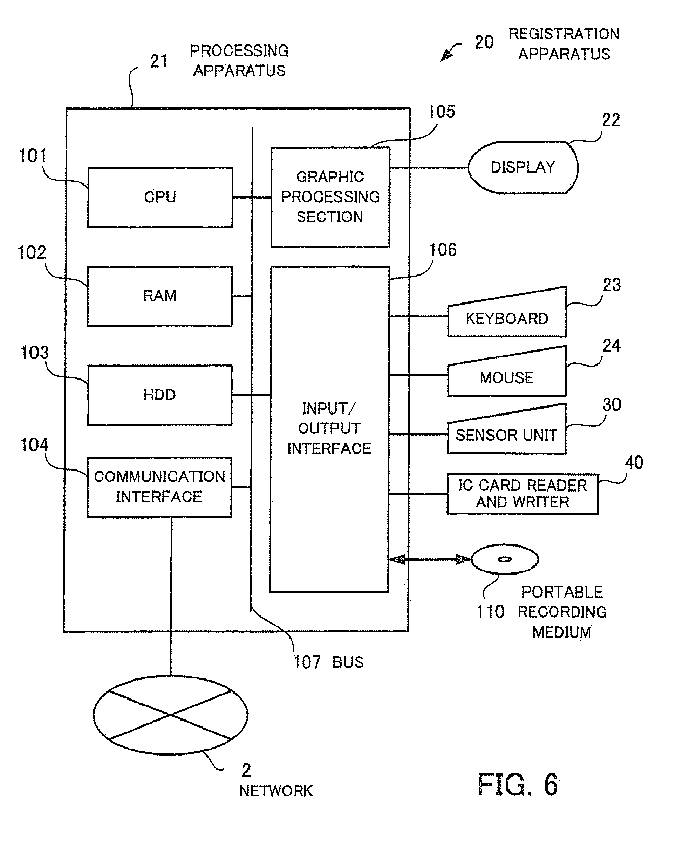

Next, an example of the hardware configuration of the registration apparatus 20 according to the present embodiment will be described with reference to FIG. 6. FIG. 6 illustrates an example of the hardware configuration of the registration apparatus according to the second embodiment.

The registration apparatus 20 comprises the processing apparatus 21, the display 22, the keyboard 23, the mouse 24, the sensor unit 30, and the IC card reader and writer 40.

The overall operation of the processing apparatus 21 is controlled by a CPU (Central Processing Unit) 101. A RAM (Random Access Memory) 102, an HDD (Hard Disk Drive) 103, a communication interface 104, a graphic processor 105, and an input/output interface 106 are connected to the CPU 101 via a bus 107.

The RAM 102 temporarily stores at least part of the program of an OS (Operating System) and application programs which the CPU 101 is caused to execute. Further, the RAM 102 stores various data required by the CPU 101 for processing. The HDD 103 stores the OS and the application programs.

The display 22 is connected to the graphic processor 105. The graphic processor 105 displays images on the screen of the display 22 according to commands from the CPU 101.

To the input/output interface 106 are connected the keyboard 23, the mouse 24, the sensor unit 30, and the IC card reader and writer 40. Further, the input/output interface 106 is configured to be connectable to a portable recording medium interface which is capable of writing information into a portable recording medium 110, and reading out information to the portable recording medium 110. The input/output interface 106 transmits signals sent from the keyboard 23, the mouse 24, the sensor unit 30, the IC card reader and writer 40, and the portable recording medium interface, to the CPU 101 via the bus 107.

The communication interface 104 is connected to the network 2. The communication interface 104 transmits and receives data between the verification apparatus 50 and the center server 60.

With the above-described hardware configuration, it is possible to realize the processing functions according to the present embodiment. Note that the verification apparatus 50 and the center server 60 as well can be realized by the same hardware configuration.

Note that each processing apparatus 21 can also be configured to include a module formed by an FPGA (Field Programmable Gate Array), a DSP (Digital Signal Processor), and so forth, and can also be configured without the CPU 101. In this case, each processing apparatus 21 is equipped with a nonvolatile memory (an EEPROM (Electrically Erasable and Programmable Read Only Memory), a flash memory, or a flash memory-type memory card, for example), and stores a firmware of the module. The firmware can be written in the nonvolatile memory via the portable recording medium 110 or the communication interface 104. Thus, the processing apparatus 21 can also update the firmware by rewriting the firmware stored in the nonvolatile memory.

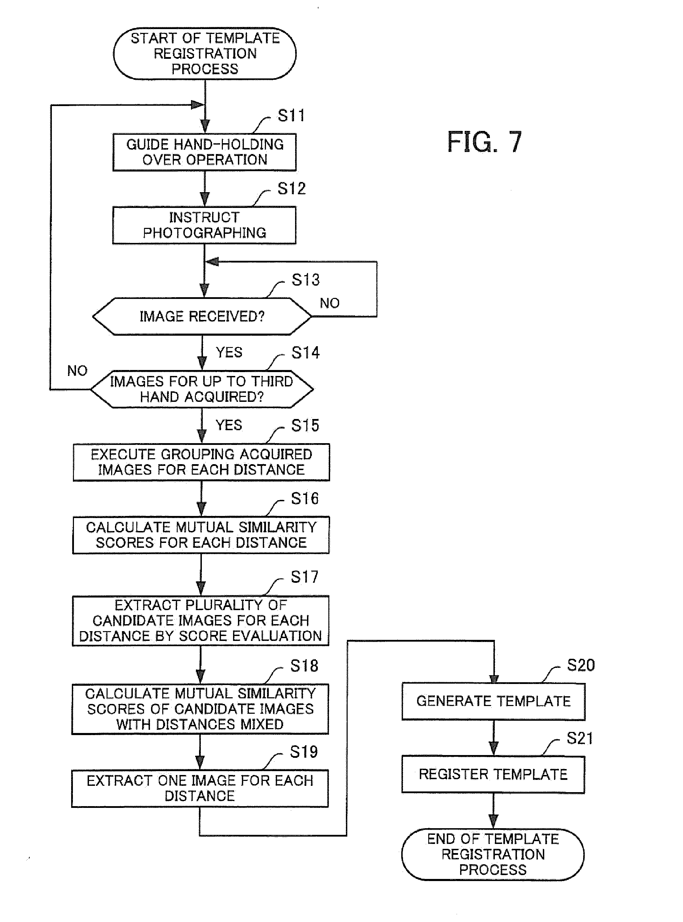

Next, a template registration process executed by the processing apparatus 21 will be described in detail with reference to FIG. 7. FIG. 7 is a flowchart of the template registration process according to the second embodiment.

The template registration process is for acquiring photographing information (photographed image) and the distance information from the sensor unit 30, and generating and registering a template.

[Step S11] The processing apparatus 21 guides a person to be registered on how to perform the palm-holding over operation 30 using an image displayed on the display 22 and a sound output from a speaker, not illustrated.

[Step S12] The processing apparatus 21 provides a photographing instruction to the sensor unit 30.

[Step S13] The processing apparatus 21 waits to receive image information and distance information associated with the image information from the sensor unit 30, and upon receipt of the image information and the distance information, the processing apparatus 21 proceeds to a step S14. The image information received by the processing apparatus 21 in this step is one acquired by a single operation of holding a hand over the sensor unit 30, performed by the person to be registered, and for example, the image information includes an average of approximately fifteen images of the living body.

[Step S14] If the processing apparatus 21 has acquired image information in an amount corresponding to three palm-holding over operations (acquired living body images for up to a third hand) from the sensor unit 30, the processing apparatus 21 proceeds to a step S15. If the processing apparatus 21 has not acquired the image information in an amount corresponding to three palm-holding over operations, the processing apparatus 21 proceeds to the step S11. Therefore, the processing apparatus 21 acquires image information amounting to approximately 45 images of the living body from the sensor unit 30.

[Step S15] The processing apparatus 21 groups the items of the image information on a predetermined distance range basis based on the associated distance information. For example, the processing apparatus 21 divides the image information of 45 images of the living body into three groups of "far", "medium", and "near" according to the distance of the sensor unit.

[Step S16] The processing apparatus 21 calculates a mutual similarity score for each group (in a manner discriminating between the groups). The mutual similarity score is an evaluation value of the similarity obtained when one of two images is set as a reference image and the other is set as a verification image. Therefore, if one group has fifteen images, the evaluation value is calculated for 15 × 14 = 210 combinations. The evaluation value is calculated by a known verification evaluation method, such as comparison of feature points, or pattern matching.

[Step S17] The processing apparatus 21 extracts image information as template candidates based on the result of evaluation of the mutual similarity scores, for each group. For example, when two images are extracted, the processing apparatus 21 extracts the two highest-evaluated verification images as image information which is to serve as template candidates. Therefore, when each two images are extracted from the three groups, the processing apparatus 21 extracts six images.

Note that the processing apparatus 21 may extract not only the highest-evaluated verification images, but also image information existing in an evaluation range different from the evaluation range of the highest-evaluated verification images, as the image information which is to serve as template candidates. For example, the image information existing in the different evaluation range may be image information which is lower in evaluation than the highest-evaluated verification images by a predetermined value. Further, the image information existing in the different evaluation range may be a verification image which is highest in evaluation except the highest-evaluated reference image and verification image.

[Step S18] The processing apparatus 21 calculates mutual similarity scores collectively (without discriminating between the groups) for the image information extracted in the step S17.

[Step S19] The processing apparatus 21 extracts the one highest-evaluated verification image in the evaluation result for each distance (group formed by grouping according to the distance range). Therefore, if the groups are three, the processing apparatus 21 extracts three images.

[Step S20] The processing apparatus 21 generates a template from the extracted three image information items. The template is obtained by performing processing on the extracted images, such as compression and deletion of information forming noise, contrast adjustment for emphasizing features, compression of the data amount (for example, calculating differences between the plurality of images), and so forth.

[Step S21] The processing apparatus 21 stores (registers) the generated template (registered template) in the storage section of the processing apparatus 21, the storage section of the center server 60, or the storage section of the IC card 41 of the user.

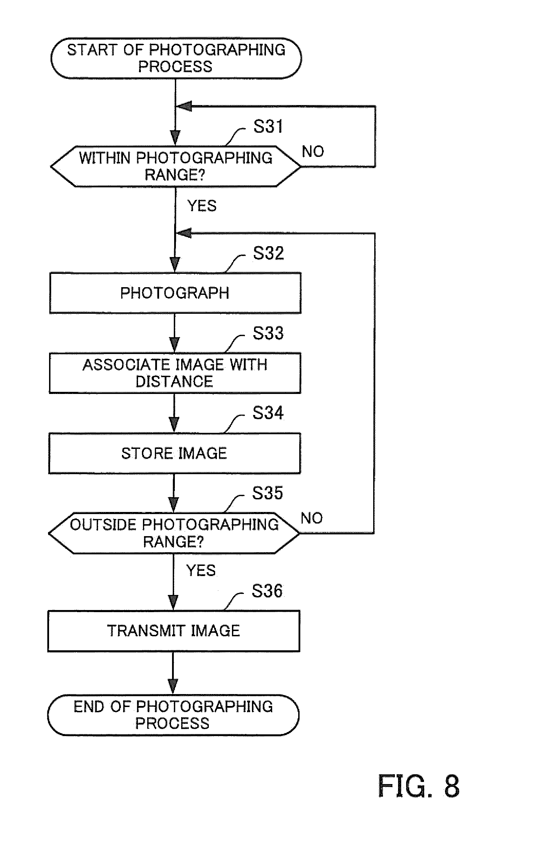

Next, a photographing process executed by the sensor unit 30 will be described in detail with reference to FIG. 8. FIG. 8 is a flowchart of the photographing process according to the second embodiment.

The photographing process is for receiving a photographing instruction from the processing apparatus 21, photographing a living body, and outputting photographing information and distance information to the processing apparatus 21.

[Step S31] The sensor unit 30 determines whether or not a living body exists in the photographing range based on the output from the distance measurement section 30c. If the living body exists in the photographing range, the sensor unit 30 proceeds to a step S32, whereas if no living body exists in the photographing range, the sensor unit 30 waits for a living body to be detected in the photographing range.

[Step S32] The sensor unit 30 photographs the living body existing in the photographing range, and acquires image information.

[Step S33] The sensor unit 30 associates distance information output from the distance measurement section 30c with the image information. The distance information is linked with (associated with) the image information e.g. by writing the distance information into a header of the image information.

[Step S34] The sensor unit 30 stores and holds the image information in the storage section 30d.

[Step S35] The sensor unit 30 determines whether or not the living body exists within the photographing range based on the output from the distance measurement section 30c. If the living body does not exist within the photographing range, the sensor unit 30 proceeds to a step S36, whereas if the living body exists in the photographing range, the sensor unit 30 proceeds to the step S32. Thus, the sensor unit 30 continuously photographs the living body existing within the predetermined range. Note that the sensor unit 30 may determine that the photographing is to be terminated when a predetermined photographing time period has elapsed or when stillness of the living body has been detected.

[Step S36] The sensor unit 30 outputs the image information stored and held in the storage section 30d to the processing apparatus 21.

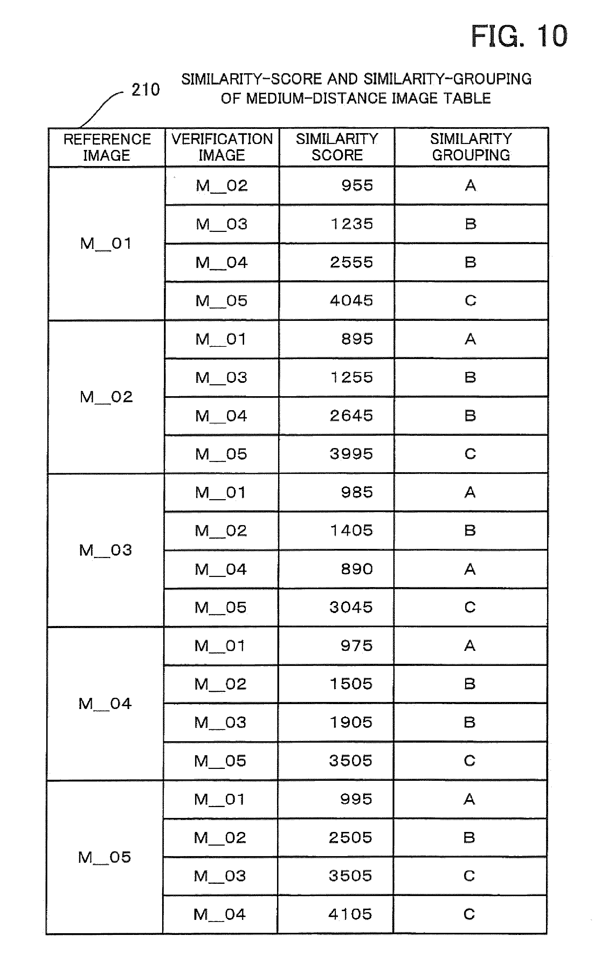

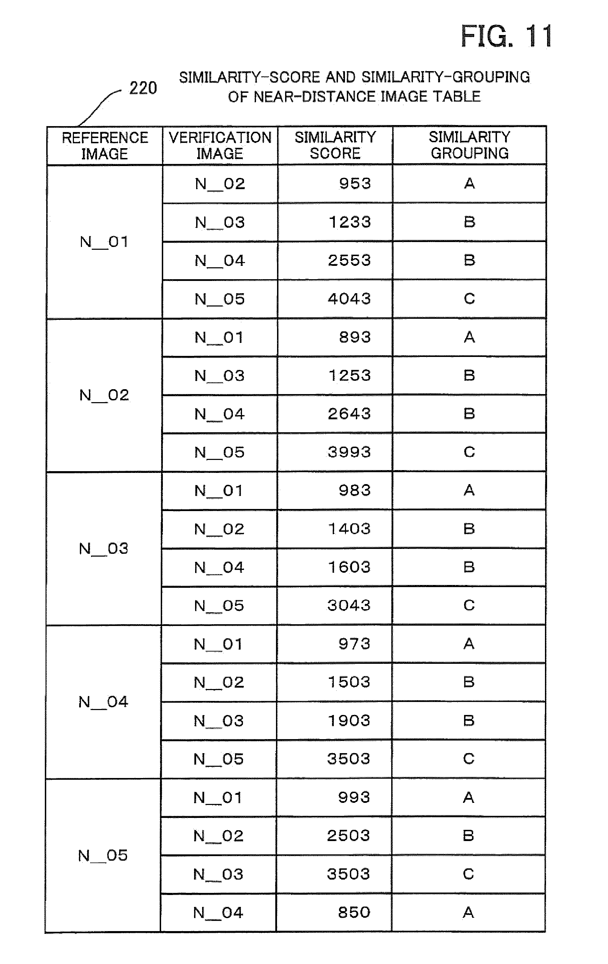

Next, the mutual evaluation of the image information will be described with reference to FIGS. 9 to 11. FIG. 9 is a table indicative of similarity scores and similarity grouping of far-distance images, according to the second embodiment. FIG. 10 is a table indicative of similarity scores and similarity grouping of medium-distance images, according to the second embodiment. FIG. 11 is a table indicative of similarity scores and similarity grouping of near-distance images, according to the second embodiment.

Although the case where one group has fifteen images has been described in the template registration process with reference to FIG. 7, by way of example, here, a case where one group has five images will be described, by way of example, for simplification of the present embodiment.

It is assumed that when the image information including total fifteen images has been acquired, the processing apparatus 21 allocates image information including five images to a group corresponding to far-distance photographing, image information including five images to a group corresponding to medium-distance photographing, and image information including five images to a group corresponding to near-distance photographing.

The group corresponding to the far-distance photographing includes images F_01, F_02, F_03, F_04, and F_05. When each of these five images is set as a reference image, and the remaining four images are set as verification images, the processing apparatus 21 obtains 5 × 4 = 20 combinations of images. The processing apparatus 21 shows mutual similarity scores calculated for these combinations on a similarity-score and similarity-grouping of far-distance image table 200. Note that even for two images of the same combination, the mutual similarity score may be different depending on which one is set as the reference image. This is because image processing before evaluation (selection and deletion of information, and characterization) differs between the reference image and the verification image.

Based on the thus obtained evaluation results, image information items having a mutual similarity score value of smaller than 1000 are classified into a similarity group A, image information items having a mutual similarity score value of not smaller than 1000 and smaller than 3000 are classified into a similarity group B, and image information items having a mutual similarity score value of not smaller than 3000 are classified into a similarity group C.

The similarity group A is a group of images evaluated as very similar, the similarity group B is a group of images evaluated as similar, and the similarity group C is a group of images evaluated as not similar. The images in the similarity groups A and B are usable for a template, but the images in the similarity group C are regarded as not usable for a template.

In this table, the top two combinations of images in the similarity score are a combination (F_02, F_01) having a similarity score of 890 and a combination (F_01, F_02) having a similarity score of 950. Therefore, from the top two combinations of images, the images F_01 and F_02 are extracted as respective images to be extracted from the group corresponding to the far-distance photographing, as template candidates.

The images to be extracted from the group corresponding to the far-distance photographing as template candidates are not limited to the top two combinations of images in the similarity score, but images may be extracted such that F_01 is extracted from the similarity group A as the highest-evaluated verification image of the group, and F_03 is extracted from the similarity group B as the highest-evaluated verification image of the group.

The group corresponding to the medium-distance photographing includes M_01, M_02, M_03, M_04, and M_05. When each of these five images is set as a reference image, and the remaining four images are set as verification images, the processing apparatus 21 obtains 5 × 4 = 20 combinations of images. The processing apparatus 21 shows mutual similarity scores calculated for these combinations on a similarity-score and similarity-grouping of medium-distance image table 210.

In this table, the top two combinations of images in the similarity score are a combination (M_03, M_04) having a similarity score of 890 and a combination (M_02, M_01) having a similarity score of 895. Therefore, from the top two combinations of images, the verification images M_01 and M_04 are extracted as respective images to be extracted from the group corresponding to the medium-distance photographing, as template candidates.

Note that as the images to be extracted from the group corresponding to the medium-distance photographing as template candidates, all images M_01, M_02, M_03, and M_04 forming the combinations may be extracted from the top two combinations of images.

Images extracted from the group corresponding to the medium-distance photographing as template candidates are not limited to the top two combinations of images in the similarity score, but images may be extracted such that M_04 is extracted from the similarity group A as the highest-evaluated verification image of the group, and M_03 is extracted from the similarity group B as the highest-evaluated verification image of the group.

The group corresponding to the near-distance photographing includes N_01, N_02, N_03, N_04, and N_05. When each of these five images is set as a reference image, and the remaining four images are set as verification images, the processing apparatus 21 obtains 5 × 4 = 20 combinations of images. The processing apparatus 21 shows mutual similarity scores calculated for these combinations on a similarity-score and similarity-grouping of near-distance image table 220.

In this table, the top two combinations of images in the similarity score are a combination (N_05, N_04) having a similarity score of 850 and a combination (N_02, N_01) having a similarity score of 893. Therefore, from the top two combinations of images, the verification images N_04 and N_01 are extracted as respective images to be extracted from the group corresponding to the near-distance photographing, as template candidates.

Note that as the images to be extracted from the group corresponding to the near-distance photographing as template candidates, all images N_01, N_02, N_04, and N_05 forming the combinations may be extracted from the top two combinations of images.

The images to be extracted from the group corresponding to the near-distance photographing as template candidates are not limited to the top two combinations of images in the similarity score, but images may be extracted such that N_04 is extracted from the similarity group A as the highest-evaluated verification image of the group, and N_03 is extracted from the similarity group B as the highest-evaluated verification image of the group.

As described above, when the respective verification images are extracted from the top two combinations of images in each distance group, the processing apparatus 21 extracts the six images of F_01, F_02, M_01, M_04, N_01, and N_04 as the template candidates. Here, 6 × 5 = 30 mutual similarity scores are calculated for these six images again. Assuming, for example, that the calculated mutual similarity scores are F_01 < F_02 < M_01 < M_04 < N_01 < N_04, the three images of F_01, M_01, and N_01 as the highest-evaluated images in the respective distance groups are extracted as the registered templates.

As described above, the processing apparatus 21 does not necessarily extract image information which is the highest in evaluation of each distance group as the registered template, but extracts image information for each distance group by causing the mutual evaluation with the other distance groups to be reflected thereon.

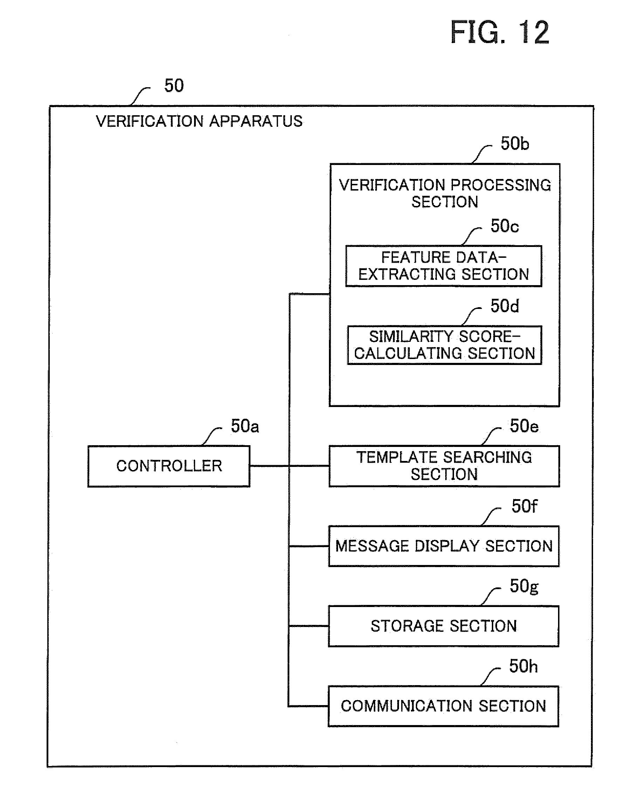

Next, verification using the template registered as above will be described. First, a description will be given of the verification apparatus 50 which realizes a process for performing verification in palm vein authentication with reference to FIG. 12. FIG. 12 illustrates the verification apparatus according to the second embodiment.

The verification apparatus 50 includes a controller 50a, a verification processing section 50b, a template searching section 50e, a message display section 50f, a storage section 50g, and a communication section 50h.

The controller 50a performs centralized control of the processing sections. The verification processing section 50b includes a feature data-extracting section 50c and a similarity score-calculating section 50d. The verification processing section 50b extracts a feature portion from image information of a living body which is a verification target by the feature data-extracting section 50c, and calculates a similarity score between the registered template and the image information from which the feature portion is extracted by the similarity score-calculating section 50d.

The template searching section 50e searches for a registered template to be used for verification. A user ID input by a user (e.g. a user ID recorded in the IC card 41 or a bank account number recorded in e.g. a magnetic stripe on a passbook or a cash card) is used as a retrieval key to search for a registered template.

The message display section 50f generates necessary messages, such as a guidance on how to perform the operation of holding a palm over a sensor unit provided in the verification apparatus 50, and a notification of whether or not the template registration is successful, and displays the generated message.

The storage section 50g stores and holds not only the image information and distance information acquired from the sensor unit provided in the verification apparatus 50 but also operation information, such as the similarity scores. The communication section 50h performs communication with the center server 60 via the network 2, and further performs communication with a required device (e.g. the sensor unit provided in the verification apparatus 50, and an IC card reader and writer provided in the verification apparatus 50).

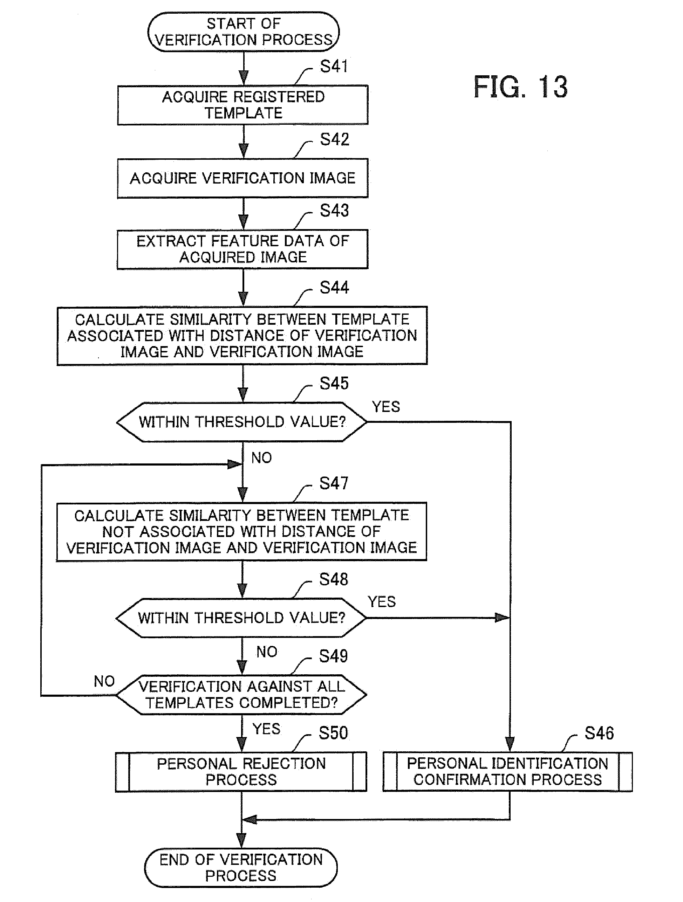

The verification process executed by the verification apparatus 50 will be described with reference to FIG. 13. FIG. 13 is a flowchart of the verification process according to the second embodiment.

[Step S41] The verification apparatus 50 receives a user ID input by a user, and notifies the center server 60 of the received user ID to acquire the registered template associated with the user ID. The verification apparatus 50 stores the acquired registered template in the storage section 50g.

If the registered template has been stored in the IC card 41 held by the user, the verification apparatus 50 acquires the registered template from the IC card 41. Further, if the verification apparatus 50 also functions as the registration apparatus, the verification apparatus 50 acquires the registered template from the storage section 50g.

At this time, if a plurality of registered templates are stored on a distance-by-distance basis, the verification apparatus 50 acquires all registered templates. Note that the verification apparatus 50 may not acquire all registered templates at a time, but may acquire the registered template for use in verification each time the verification is executed.

[Step S42] The verification apparatus 50 guides the user on the palm-holding over operation with a proper message display by the message display section 50f. The verification apparatus 50 acquires the image information of the living body for use in verification and the distance information (hereinafter referred to as the verification target distance information) associated with the image information from the sensor unit provided in the verification apparatus 50.

[Step S43] The verification apparatus 50 extracts feature data (feature portion) from the image information of the living body as a verification target by the feature data-extracting section 50c.

[Step S44] The verification apparatus 50 calculates a degree of similarity between a registered template associated with the verification target distance information and the image information from which the feature data has been extracted (calculation of a similarity score).

[Step S45] The verification apparatus 50 determines whether or not the result of calculation of similarity is within a predetermined threshold value. If the result of calculation of similarity is within the predetermined threshold value, the verification apparatus 50 proceeds to a step S46, whereas if the result of calculation of similarity is not within the predetermined threshold value, the verification apparatus 50 proceeds to a step S47. The predetermined threshold value is a value set in advance based on allowance settings of a false rejection rate and a false acceptance rate for each installation environment of the verification apparatus 50.

[Step S46] The verification apparatus 50 executes a personal identification confirmation process, followed by terminating the verification process. The verification apparatus 50 displays a personal identification confirmation message by the message display section 50f, and allows the user to execute processing to be executed after personal authentication.

[Step S47] The verification apparatus 50 calculates a similarity between a registered template which is not associated with the verification target distance information and the image information from which the feature data has been extracted (calculation of a similarity score).

[Step S48] The verification apparatus 50 determines whether or not the result of calculation of similarity is within a predetermined threshold value. If the result of calculation of similarity is within the predetermined threshold value, the verification apparatus 50 proceeds to the step S46, whereas if the result of calculation of similarity is not within the predetermined threshold value, the verification apparatus 50 proceeds to a step S49.

[Step S49] The verification apparatus 50 determines whether or not verification of the image information from which the feature portion has been extracted against all of the registered templates has been completed. If verification against all of the registered templates have been completed, the verification apparatus 50 proceeds to a step S50, whereas if verification against all of the registered templates have not been completed, the verification apparatus 50 proceeds to the step S47.

[Step S50] The verification apparatus 50 executes a personal rejection process, followed by terminating the verification process. The verification apparatus 50 displays a personal rejection message by the message display section 50f, and does not allow the user to execute processing to be executed after personal authentication.

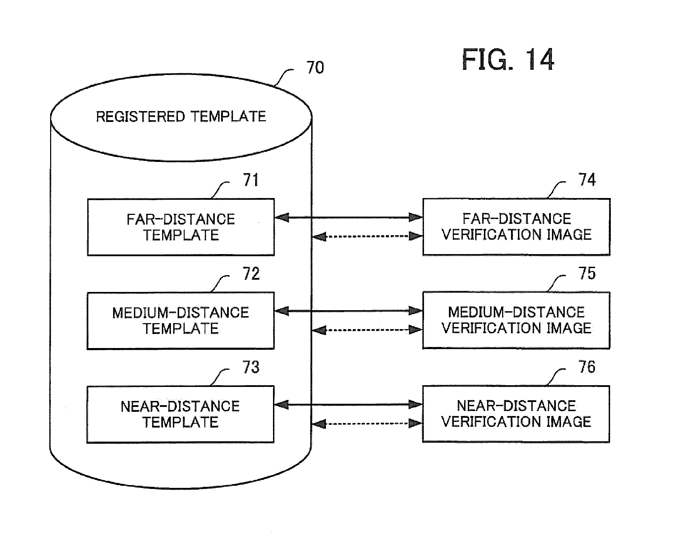

Correspondence between the registered templates and verification images as described above will be described with reference to FIG. 14. FIG. 14 illustrates registered templates and verification images according to the second embodiment.

First, the verification apparatus 50 performs verification using a registered template associated with the verification target distance information (correspondence indicated by solid lines). Then, if the verification using the registered template associated with the verification target distance information is not successful, the verification apparatus 50 further performs verification using registered templates which are not associated with the verification target distance information (correspondence indicated by broken lines).

For example, a far-distance template 71, a medium-distance template 72, and a near-distance template 73 have been registered in a registered template 70. At this time, a far-distance verification image 74 is verified against the far-distance template 71, and if this verification is not successful, the far-distance verification image 74 is verified against the medium-distance template 72 and the near-distance template 73. Similarly, a medium-distance verification image 75 is verified against the medium-distance template 72, and if this verification is not successful, the medium-distance verification image 75 is verified against the far-distance template 71 and the near-distance template 73. Similarly, a near-distance verification image 76 is verified against the near-distance template 73, and if this verification is not successful, the near-distance verification image 76 is verified against the far-distance template 71 and the medium-distance template 72.

As a result, even when the user has not properly performed a palm-holding over operation in part of the template registration process, it is possible to increase the chances to properly perform verification (smoothly perform authentication). Alternatively, even for users who do not properly perform the palm-holding over operation in part of the process for a palm-holding over operation, it is possible to increase the chances to properly perform verification.

Although in the case where the verification using a registered template associated with the verification target distance information is not successful, the verification apparatus 50 performs verification using registered templates which are not associated with the verification target distance information, the verification may be performed such that a verification image is verified against all of the registered templates from the start.

Further, the verification processing section 50b may be provided not in the verification apparatus 50, but in the center server 60 or the IC card 41. In this case, the verification apparatus 50 notifies the center server 60 or the IC card 41 of the user ID and image information of a living body which is a verification target (including associated distance information), and acquires a result of verification.

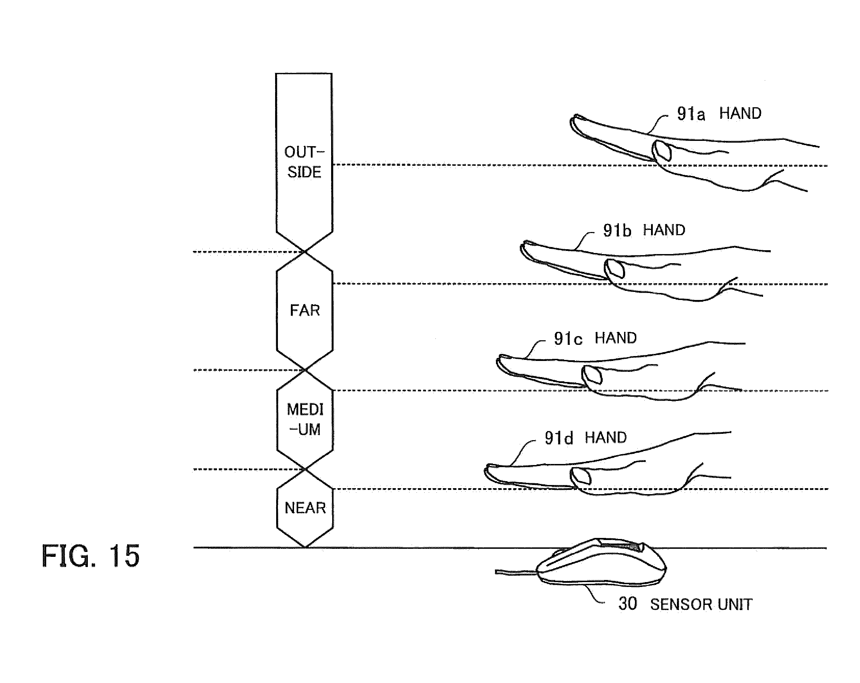

Next, a third embodiment will be described with reference to FIG. 15. FIG. 15 illustrates how template registration according to the third embodiment is performed. The third embodiment differs from the second embodiment in that an actual distance is unequally divided and images are grouped according to the divided distances. As for the same arrangement as that of the second embodiment, a description is given using the same reference numerals.

### [Third Embodiment]

The sensor unit 30 includes a distance measurement sensor and photographs a palm within a predetermined distance from the sensor unit 30. When the photographing range of the sensor unit 30 is set to 20 mm to 80 mm, the distance of 60 mm is equally divided in the second embodiment, but is unequally divided in the third embodiment.

That is, the threshold used for grouping has reference values set in advance such that a range from 20 mm to 30 mm is set as "near", a range from 30 mm to 50 mm is set as "medium", and a range from 50 mm to 80 mm is set as "far". With this setting, when the images of the palm are obtained using a fixed photographing speed, even if the user reduces the speed of motion of his/her hand as the hand is moved from far to near the sensor unit 30 as indicated by hands 91a, 91b, 91c, and 91d, it is possible to obtain approximately the same number of images of the palm for each distance.

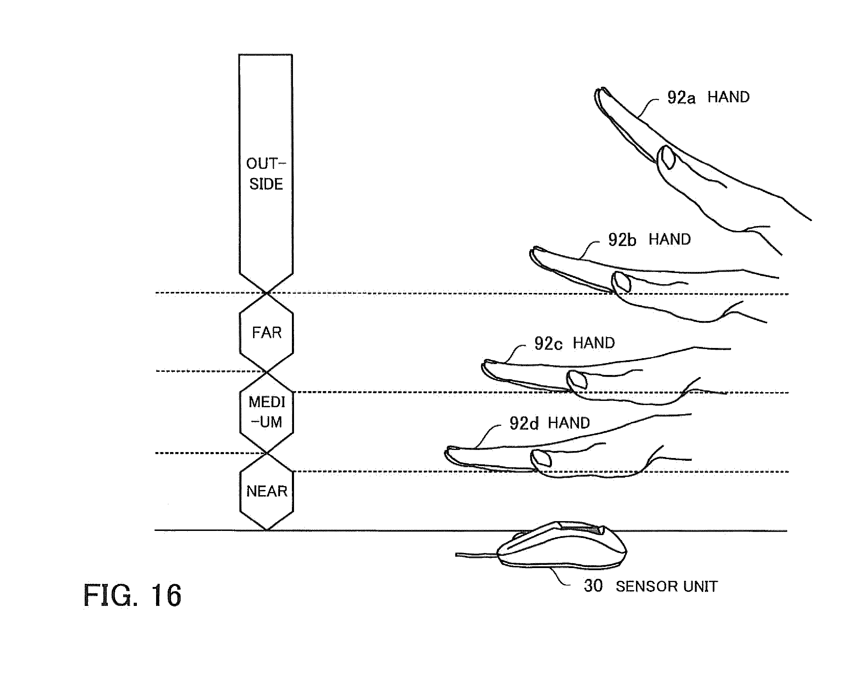

Next, a fourth embodiment will be described with reference to FIG. 16. FIG. 16 illustrates template registration according to the fourth embodiment.

The fourth embodiment differs from the third embodiment in that grouping is performed by setting the reference values based on the photographed images. As for the same arrangement as that of the third embodiment, a description is given using the same reference numerals.

### [Fourth Embodiment]

The sensor unit 30 includes a distance measurement sensor and photographs a palm within a predetermined distance from the sensor unit 30. When the photographing range of the sensor unit 30 is set to 20 mm to 80 mm, an image of the palm at the height of 80 mm is not necessarily obtained. This is because the palm is not necessarily moved down toward the sensor unit 30 from above, but is sometimes moved toward the sensor unit 30 with a motion in a horizontal direction or a front-rear direction.

In this case, when the sensor unit 30 has photographed the palm, within a photographing range of 20 mm to 50 mm, the plurality of photographed images are divided into three groups according to the distance. Therefore, the threshold value used for grouping has the reference values set after photographing such that a range from 20 mm to 30 mm is "near", a range from 30 mm to 40 mm is "medium,", and a range from 40 mm to 50 mm is "far".

That is, with this setting, when the images of the palm are obtained at a fixed photographing speed, even if the hand is moved closer to the sensor unit 30 in an undesirable manner as indicated by hands 92a, 92b, 92c, and 92d, it is possible to obtain the images of the palm for each distance.

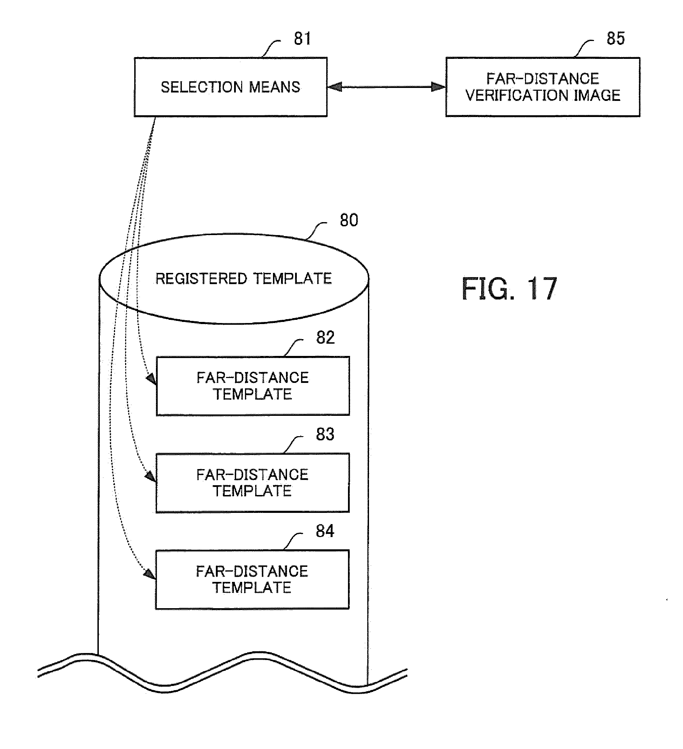

Next, a fifth embodiment will be described with reference to FIG. 17. FIG. 17 illustrates registered templates and a verification image according to the fifth embodiment. The fifth embodiment differs from the second embodiment in that a plurality of templates are registered for each distance. As for the same arrangement as that of the second embodiment, a description is given using the same reference numerals.

### [Fifth Embodiment]

A registration template 80 includes a plurality of far-distance templates (a far-distance template 82, a far-distance template 83, and a far-distance template 84), a plurality of medium-distance templates (not illustrated), and a plurality of near-distance templates (not illustrated). A selection means 81 may be provided in the verification apparatus 50, or in an apparatus which stores the registered templates, such as the center server 60.

First, the verification apparatus 50 obtains a registered template associated with the verification target distance information from the selection unit 81 to perform verification (correspondence indicated by a solid line). The selection unit 81 selects one registered template from the plurality of registered templates associated with the verification target distance information as a verification target (indicated by broken lines).

Therefore, a far-distance verification image 85 is first verified against one of the far-distance template 82, the far-distance template 83, and the far-distance template 84, and then is verified against the rest of the far-distance templates if the first verification is not successful. The verification of medium-distance images and that of near-distance images are performed in the same manner.

Note that the registered template may be randomly selected by the selection unit 81, or may be sequentially selected. Further, the order of selection may be changed according to the verification result.

Note that the number of templates registered in the registered template 80 may be set according to various restrictions of the authentication system 1 (e.g. storage capacity and processing capacity).

As a result, even when the user has not properly performed a palm-holding over operation in part of the template registration process, it is possible to increase the chances to properly perform verification (smoothly perform authentication) without repeating the palm-holding over operation. Alternatively, even for users who do not properly perform the palm-holding over operation in part of the process for a palm-holding over operation, it is possible to increase the chances to properly perform verification without repeating the palm-holding over operation.

Note that the processing functions of the above-described embodiments can be realized by a computer. In this case, there is provided a program describing the details of processing of the functions which the registration apparatus 20, the center server 60, and the verification apparatus 50 are to have. By executing the program by the computer, the processing functions described above are realized on the computer. The program describing the details of processing can be recorded in a computer-readable storage medium (including a portable recording medium). Examples of the computer-readable recording medium include a magnetic recording device, an optical disk, a magneto-optical recording medium, and a semiconductor memory. Examples of the magnetic recording device include a hard disk drive (HDD), a flexible disk (FD), and a magnetic tape. Examples of the optical disk include a DVD (Digital Versatile Disk), a DVD-RAM, a CD-ROM, a CD-R (Recordable)/RW (ReWritable). Examples of the magneto-optical recording medium include an MO (Magneto-Optical Disc).

In case of distributing programs, for example, portable recording mediums, such as DVD, CD-ROM or the like in which the program is recorded are marketed. Further, it is also possible to store the program in a storage device of a server computer, and transfer the program from the server computer to the other computer via a network.

The computer which carries out the program stores, for example, the program which is recorded in the portable recording medium, or is transferred from the server computer in the storage device thereof. Then, the computer reads out the program from the storage device thereof, and carries out the processes according to the program. Note that the computer is also capable of directly reading out the program from the portable recording medium, and carrying out the processes according to the program. Further, the computer is also capable of carrying out the processes according to the program which is received, each time the program is transferred from the server computer.

Note that the above-described embodiment can be subjected to various modifications and alterations without departing from the gist of the present invention.

Further, since numerous modifications and changes will readily occur to those skilled in the art, it is not desired to limit the above-described embodiment to the exact construction and applications shown and described.

The foregoing is considered as illustrative only of the principles of the present invention. Further, since numerous modifications and changes will readily occur to those skilled in the art, it is not desired to limit the invention to the exact construction and applications shown and described, and accordingly, all suitable modifications and equivalents may be regarded as falling within the scope of the invention in the appended claims and their equivalents.

### Reference Signs List

- 1: authentication system
- 2: network
- 10: registration apparatus
- 10a: image information acquisition means
- 10b: distance information acquisition means
- 10c: template candidate extraction means
- 10d: template extraction means
- 20: registration apparatus
- 20a: controller
- 20b: image grouping processing section
- 20c: verification processing section
- 20d: candidate image-extracting section
- 20e: template image-extracting section
- 20f: template generation section
- 20g: template registration section
- 20h: message display section
- 20i: storage section
- 20j: communication section
- 21: processing apparatus
- 30: sensor unit
- 30a: controller
- 30b: photographing section
- 30c: distance measurement section
- 30d: storage section
- 30e: communication section
- 40: IC card reader and writer
- 41: IC card
- 50: verification apparatus
- 50a: controller
- 50b: verification processing section
- 50c: feature data-extracting section
- 50d: similarity score-calculating section
- 50e: template searching section
- 50f: message display section
- 50g: storage section
- 50h: communication section
- 60: center server

## Claims

1. A registration program for registering a template for use in biometric authentication, the registration program causing a computer to execute a procedure comprising:
acquiring, from a sensor unit which is capable of outputting image information of a photographed living body and distance information acquired when photographing the living body, a plurality of items of the image information;
acquiring the distance information associated with each item of the image information from the sensor unit;
grouping the plurality of items of the image information according to the distance information associated with each item of the image information, and mutually evaluating the image information on a group-by-group basis according to the distance information to thereby extract an item of the image information, which is to serve as a template candidate; and
mutually evaluating extracted items of the image information to thereby extract an item of the image information, which is to serve as a template.

2. The registration program according to claim 1, wherein when acquiring the plurality of items of the image information from the sensor unit, the living body being moved closer to the sensor unit is continuously photographed, and the plurality of items of the image information generated by the sensor unit from a single registration operation are acquired.

3. The registration program according to claim 2, wherein when acquiring the plurality of items of the image information from the sensor unit, the plurality of items of the image information generated by the sensor unit from a plurality of registration operations are acquired.

4. The registration program according to claim 1, wherein the grouping according to the distance information is performed based on distance criteria between the sensor unit and the living body, set in advance.

5. The registration program according to claim 1, wherein the grouping according to the distance information is performed based on distance criteria generated from the distance information associated with the plurality of items of the image information generated by the sensor unit.

6. The registration program according to claim 1, wherein the grouping according to the distance information is performed based on the number of the plurality of items of the image information generated by the sensor unit.

7. The registration program according to any one of claims 1 to 6, wherein when extracting the item of the image information, which is to serve as a template candidate, a plurality of items of the image information which are highest-evaluated in the mutual evaluation are extracted for each group associated with the distance information.

8. The registration program according to claims 1 to 6, wherein when extracting the item of the image information, which is to serve as a template candidate, items of the image information which are in respective different evaluation zones in the mutual evaluation are extracted for each group associated with the distance information.

9. A registration apparatus that registers a template for use in biometric authentication, comprising:
image information acquisition means for acquiring, from a sensor unit which is capable of outputting image information of a photographed living body and distance information acquired when photographing the living body, a plurality of items of the image information;
distance information acquisition means for acquiring the distance information associated with each item of the image information from the sensor unit;
template candidate extraction means for grouping the plurality of items of the image information according to the distance information associated with each item of the image information, and mutually evaluating the image information on a group-by-group basis according to the distance information to thereby extract an item of the image information, which is to serve as a template candidate; and
template extraction means for mutually evaluating extracted items of the image information to thereby extract an item of the image information, which is to serve as a template.

10. A method of registering a template for use in biometric authentication, comprising:
acquiring, from a sensor unit which is capable of outputting image information of a photographed living body and distance information acquired when photographing the living body, a plurality of items of the image
information;
acquiring the distance information associated with each item of the image information from the sensor unit;
grouping the plurality of items of the image information according to the distance information associated with each item of the image information, and mutually evaluating the image information on a group-by-group basis according to the distance information to thereby extract an item of the image information, which is to serve as a template candidate; and
mutually evaluating extracted items of the image information to thereby extract an item of the image information, which is to serve as a template.
